# EUROPEAN PATENT APPLICATION

(11) **EP 3 245 942 A1**
(43) Date of publication of application: **22.11.2017**
(21) Application number: 16170246.9
(22) Date of filing: 19.05.2016
(51) Int. Cl.: A61B 5/00, A61B 5/0205, A61B 5/11

(54) **LOCATION DEVICE FOR IDENTIFYING LOCATION(S) FOR PHYSIOLOGICAL MEASUREMENTS AND METHODS USING THE SAME**

(71) Applicant: Nokia Technologies Oy, 02610 Espoo (FI)
(72) Inventor: SALO, Antti Oskari, 08500 Lohja as. (FI); LASAROV, Harri Aukusti, 02280 Espoo (FI)
(74) Representative: Nokia EPO representatives

(57) **Abstract**

Provided are location devices that identify the location for measuring physiological conditions, methods of operating the device, and computer program products for use with the device. The location device provides improved reliability in measurement data since it consistently provides the location for the measurements. In the context of a location device, an apparatus is provided that includes a structure configured to be attached to the skin of a user and configured to identify a location for physiological measurement. The structure has a shape configured to correspond to a portion of the user that is in a predefined positional relationship to the location for the physiological measurement. As such, the structure is configured to engage the portion of the user in order to identify the location for the physiological measurement. The location device may include one or more sensors for measuring physiological conditions or may work in conjunction with one or more sensors.

## Description

### FIELD

An example embodiment of the present invention relates to a location device, method of using the same, and computer program product for the same. The location device identifies the location for the measurement of physiological conditions of a user. The location device may include one or more sensors for measuring physiological conditions of a user and/or may be used with an external sensor to measure physiological conditions of a user.

### BACKGROUND

Difficulties can arise in identifying the optimal location for measuring physiological conditions of a patient, particularly when the patient is the one taking the measurement. Without the help of a medical professional, the patient may have difficulty locating the optimal location for measurement and even when located, due to changes in position and movement, the patient may have difficulty maintaining the optimal position for measurement. The failure to identify and maintain the optimal location for measurement may reduce the quality of the measurement data. In addition, the sensor may suffer from inadequate or compromised sensor measurement readings and data due to relative three axis movement between the sensor and the patient's skin. In addition, a photodetector could be exposed to unwanted ambient light or light leakage due to movement. Optical sensors are particularly sensitive to changes in position, for instance, when monitoring the pulsation of blood vessels.

Reliable measuring (e.g., photoplethysmography (PPG), skin temperature, electro dermal activity (EDA), bio impedance, electromyography (EMG), electrocardiography (ECG) and electroencephalography (EEG)) of bio signals and vital signs is preferably obtained at certain locations on a patient. Optical sensors are particularly sensitive to the measuring position when monitoring the pulsation in the chest region. Even millimeter shifts from the optimum position can decrease the quality of PPG signals (particularly peripheral oxygen saturation SpO2) dramatically as well as make the comparison of data difficult due to the variation.

Medical professionals are trained to find the optimum position for measurement. However, when a patient leaves the hospital or medical center, remote monitoring of the physiological conditions may still be needed. Patients often have difficulty positioning the sensor in the correct location. Identification may be made even more difficult when a patient moves (e.g., posture changes, limb movement or even breathing) and/or is subjected to external interferences, causing the patient to question the location of the sensor.

### SUMMARY

Example embodiments of the present invention provide location devices, methods of using such devices, and computer program products for use with such devices. Location devices in accordance with example embodiments of the present invention may help provide improved identification of the locations for physiological measurements and thereby provide more accurate and reliable measurements of physiological conditions.

An example embodiment of the present invention provides location devices that are used on the skin of a user to identify a location for measuring physiological conditions of the user. The location device includes a structure that is positioned on the skin of the user and thereby identifies the location for physiological measurements. In some example embodiments, the location device includes sensors for obtaining the physiological measurements, while in other embodiments, the location device is physically separate from the sensor.

The location device may be designed to be positioned at certain locations on the user's body such that the patient can easily re-position the device at the location for measuring the relevant physiological condition when needed. For instance, the device may be designed to fit over the bone of the user, such as a chest bone, where the optimal location for physiological measurement is located, such that the user can then easily re-position the device over the bone and obtain the physiological measurement. The location device may include a sensor and related circuitry and/or electronics for measuring the relevant physiological condition and may be user activated when the relevant physiological measurement is needed. The location device may work with a second device that includes a sensor and related circuitry for measuring one or more physiological conditions and/or external conditions. The second device including the sensor can be matched to the location device when measurements are needed.

In some embodiments, the device may be attached to the skin of the user by a medical professional. For instance, the medical professional may attach the device to the skin of the user at the location for the relevant physiological measurement. When the patient later proceeds to take the physiological measurement, the patient has the device already attached and in place, such that the patient can easily locate the location for measurement and obtain the applicable measurement.

The physiological readings may thereby be more accurate and reliable due to the consistency of the location of measuring. Further, the location device allows for patients to be able to obtain accurate and reliable data without the immediate assistance of a medical professional and with added confidence in the data. The patient can obtain the physiological measurements away from the hospital or medical center, such as at home. The patient is also more likely to continue monitoring the relevant physiological condition because he/she is confident that the measurements are being taken in the correct position.

The location device may be made with the sensor and related circuitry permanently attached to the device, while in some embodiments, the location device is made to temporarily connect or join to the sensor and related circuitry. For instance, the device may be configured such that the sensor housing adjoins the device when the physiological measurement is needed and can then be disconnected when the physiological measurement is completed, leaving the location device on the skin of the user. In some embodiments, the sensor and sensor housing are an integral part of the location device.

The location device of some embodiments is easy to manufacture and can be made to include or work with various sensors or electrodes for measuring physiological conditions of the user as well as external conditions of the environment. The device can be made with flexible, cushioning, and/or stretchable material to provide user comfort.

According to at least some but not necessarily all examples of the disclosure there is provided an apparatus comprising a structure configured to identify a location for a physiological measurement on skin of a user. The structure of the location device may have a shape configured to correspond to a portion of the user that is in a predefined positional relationship to the location for the physiological measurement such that the structure is configured to engage the portion of the user in order to identify the location for the physiological measurement. The structure includes a first surface and a second surface with the first surface being configured to be adjacent to the skin of the user. In some embodiments, the structure may comprise a layer of ink configured to identify a location for physiological measurement on the skin of the user. In certain embodiments, the structure may comprise a portion configured to fit over a chest bone of a user.

In some embodiments of the present disclosure, the apparatus comprises a structure configured to be read by a sensor and convey information regarding the physiological measurement and/or the user. The physiological measurement may be one or more of the following: heart rate, heart rate variability, arrhythmia, blood pressure, blood oxygen, blood glucose, humidity, temperature, galvanic skin response, or skin moisture. In some embodiments, the structure may be configured to connect to a sensor and/or sensor housing for the physiological measurement.

The apparatus may be configured for wearable use on the user's torso or appendage, e.g. limb, and may comprise an attachment mechanism. The apparatus may be configured for any suitable part of the user's body. For example, the apparatus may be configured as a patch or tattoo that attaches to the skin of a user or may be configured as a wrist device that wraps around and can be worn on a user's wrist or other appendage such as the user's foot. The attachment mechanism may comprise one or more of a belt, a snap, a tie, or an adhesive based attachment mechanism.

The apparatus may comprise one or more sensors, where the one or more sensors may be configured to monitor the user and/or the external environment. For instance, the apparatus may comprise a second sensor configured to monitor the user and/or external environment or may work in conjunction with a second sensor.

In addition, in some embodiments, the device may comprise one or more of: a sensor, a power supply, electronics, and circuitry. The power supply, electronics, and circuitry may be configured to control one or more sensors. The circuitry may be flexible circuitry and may be made integral to the device.

In certain embodiments, the apparatus may comprise a user actuation section. For instance, in some embodiments, the apparatus may comprise at least one sensor configured to detect user actuation of a part of the apparatus. The sensor signal from the at least one sensor configured to detect user actuation of a part of the apparatus may be configured to control the apparatus.

According to at least some but not necessarily all examples of the disclosure there is provided a system comprising the apparatus as described above and one or more sensors configured to measure one or more physiological conditions of the user. In this embodiment, the one or more sensors is physically separable from the apparatus.

According to at least some but not necessarily all examples of the disclosure there is provided a method for operating the apparatus, comprising: detecting a user actuation of the apparatus; and controlling operation of the apparatus in dependence on the detected user actuation.

According to at least some but not necessarily all examples of the disclosure there is provided a computer program that, when performed by at least one processor, causes the above method to be performed.

According to at least some but not necessarily all examples of the disclosure there is provided a computer program product that includes a non-transitory computer readable medium encoded with instructions that, when performed by at least one processor, causes the above method to be performed.

These embodiments of the present invention and other aspects and embodiments of the present invention are described further herein and will become apparent upon review of the following description taken in conjunction with the accompanying drawings.

### BRIEF DESCRIPTION OF THE SEVERAL VIEWS OF THE DRAWINGS

Having thus described example embodiments of the invention in general terms, reference will now be made to the accompanying drawings, which are not necessarily drawn to scale, and wherein:
FIG. 1 schematically illustrates an example of an apparatus according to an example embodiment of the present disclosure;
FIG. 2 schematically illustrates an example of an apparatus including a sensor according to an example embodiment of the present disclosure;
FIG. 3 schematically illustrates an example of an apparatus that is used with an external sensor according to an example embodiment of the present disclosure;
FIG. 4 schematically illustrates an example of an apparatus according to an example embodiment of the present disclosure;
FIG. 5 schematically illustrates an example of an apparatus according to another example embodiment of the present disclosure;
FIG. 6 schematically illustrates another example of an apparatus including an identifier according to an example embodiment of the present disclosure;
FIG. 7 schematically illustrates a user with a plurality of example apparatus according to an example embodiment of the present disclosure;
FIG. 8 illustrates an example of a controller for an example apparatus according to an embodiment of the present disclosure; and
FIG. 9 illustrates a method according to an example embodiment of the present disclosure.

### DETAILED DESCRIPTION

The present invention now will be described more fully hereinafter with reference to the accompanying drawings, in which some, but not all embodiments of the invention is shown. Indeed, the invention may be embodied in many different forms and should not be construed as limited to the embodiments set forth herein; rather, these embodiments are provided so that this disclosure will satisfy applicable legal requirements. Like numbers refer to like elements throughout.

As used in the specification and in the appended claims, the singular forms "a", "an", and "the" include plural referents unless the context clearly indicates otherwise. For example, reference to "a sensor" includes a plurality of such sensors, unless the context clearly indicates otherwise.

As used in the specification and in the appended claims, reference to "on" includes both embodiments in which a component is disposed directly on another component as well as embodiments in which one or more intervening layers or elements are disposed between the components.

As used in the specification and in the appended claims, reference to "sensor housing" includes one more sensors and may include additional components such as electronics, circuitry and power supply that may support the one or more sensors.

As used in the specification and in the appended claims, reference to "wearer" refers to the individual wearing the disclosed device and may also be referred to as "patient" or "user."

As used in the specification and in the appended claims, reference to "user actuation" refers to initiation of any action in the device due to the input of a user. For example, user actuation may refer to user touch or voice control (e.g., by pressing a button, squeezing the device by the user, talking to the device, etc.) that initiates one or more actions in the device (e.g., measuring a physiological condition of the user). As used herein, "user manipulation" refers to physical manipulation of the device by the user that may or may not trigger an action in the device.

In an example embodiment, the location device enables more accurate and reliable physiological measurements. In example embodiments of the present disclosure, the apparatus or location device comprises a structure configured to be positioned on the skin of a user. As described, the structure has a shape configured to correspond to a portion of the user that is in a predefined positional relationship to the location for the physiological measurement, such as by overlying or being immediately adjacent the optimal location for the physiological measurement, such that the structure is configured to engage the portion of the user in order to identify the location for the physiological measurement. The structure comprises a first surface and a second surface with the first surface configured to be adjacent to the skin of the user. The structure is configured to identify a location for measuring a physiological condition of the user. The apparatus may also include one or more sensors and/or circuitry and can be attached to the user temporarily. Various configurations of the device, such as those described in detail below, may be used to improve the identification of locations for physiological measurements.

In certain embodiments, the device may be designed to fit on a certain part of the body of the user such that the device can be removed and easily re-positioned at that particular part of the body ensuring the optimal location is repeatedly obtained. The device may include or be configured to work with a sensor that measures the physiological condition of the user. The device may also have circuitry for operating the sensor and/or receiving/transferring data regarding the user and/or physiological condition. The device may contain readable information regarding the user, physiological condition, or other relevant information.

In some embodiments, the device may be temporarily attached to the user and may be easily removable such as by peeling off the device, washing off the device, un-snapping/clipping/zipping/strapping the device, or otherwise removing the device from the user's skin.

An example embodiment of the apparatus according to the present disclosure will now be described with reference to the figures. Many modifications of the device and components of the example embodiments will come to mind to one skilled in the art to which these inventions pertain having the benefit of the teachings presented in the present descriptions and the associated drawings. Therefore, it is to be understood that the inventions are not to be limited to the specific embodiments disclosed or specific drawings of those embodiments and that modifications and other embodiments are intended to be included within the scope of the appended claims. Similar reference numerals are used in the figures to designate similar features. For clarity, all reference numerals are not necessarily displayed in all figures.

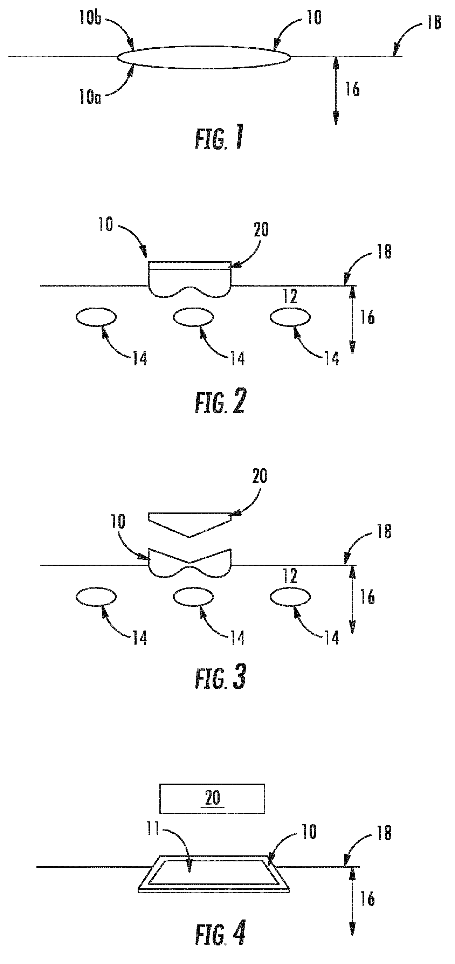

FIG. 1 schematically illustrates an example of an apparatus according to an example embodiment of the present disclosure. In particular, FIG. 1 provides a cross-sectional view of the apparatus positioned on the user's skin. The device 10 illustrated in FIG. 1 is positioned on the surface 18 of the body 16 of the user. The device 10 includes a structure having a first surface 10a and a second surface 10b, which may be opposite the first surface 10a and may consequently face the opposite direction. The first surface 10a is configured to be adjacent to the surface 18 of the body 16 of the user. As described herein, the structure, including the first surface 10a, has a shape configured to correspond to a portion of the user that is in a predefined positional relationship to the location for the physiological measurement, such as by overlying or being immediately adjacent the optimal location for the physiological measurement, such that the structure is configured to engage the portion of the user in order to identify the location for the physiological measurement. The device may be attached to the skin by any suitable means, such as adhesive, straps, snaps, belt, or combinations thereof. For instance, the device may include an adhesive layer that adheres the device to the skin of the user. In some embodiments, the device is positioned on the skin of the user and the complement structure of the device to the part of the body allows the device to maintain its relative position on the user.

In some embodiments, the device may comprise pigments and/or dyes applied to the skin. For instance, the device may be transferred to the skin of the user by applying a substrate containing ink to the skin such that the ink transfers to the skin and the substrate may be peeled off the skin. This transfer may be initiated by application of water or other liquid. The substrate, such as paper, can then be discarded. In some embodiments, pigments or dyes may be applied to the skin by a syringe or cone-shaped device, such as that seen with henna tattoos. Pigments or dyes may be applied to the skin of the user by any suitable method. In certain embodiments, the location device comprises a structure comprising a layer of pigment or dye positioned on the skin of the user, the layer having a first surface and a second surface with the first surface adjacent to the skin of the user.

Any suitable attachment mechanism may be employed to temporarily, semi-permanently and permanently attach the apparatus to the user, whether partially enclosing the torso or an appendage of the user or not. Such mechanisms may include, for example: belts, bands, bracelets, straps (e.g., to be tied), bandages, stickers, tapes, plaster, fastening means (e.g., snaps), adhesive means, and/or tattoos. The mechanism may be adjustable, stretchable and conformable to a user to ensure an appropriate level of tightness of fit. For instance, the attachment mechanism may include a fastening device to secure and tighten the apparatus around a user's appendage or torso. For example, a hook and loop based fastening strap may be used in this regard. Alternatively, other fastening and/or clasping means may be used.

FIG. 2 schematically illustrates an example of an apparatus including a sensor according to example embodiments of the present disclosure. In particular, FIG. 2 provides a cross-sectional view of the apparatus positioned on the user's skin. In the embodiment of FIG. 2, the location device 10 includes a sensor housing 20. The device 10 is positioned on the surface 18 of the body 16 of the user and includes a first surface 10a and a second surface 10b. FIG. 2 also schematically illustrates tissue 12 and bone 14 located in the body 16 of the user. The device 10 in FIG. 2 is configured to complement the part of the body where it is intended to be positioned. For instance, the device in FIG. 2 is shaped to fit around the underlying bone 14 in the body 16 of the user. By configuring the device in such manner, the device can be positioned over the particular part of the body and ensure that the physiological condition will be measured at the optimal location. The shape of the device may also improve the stability of the device once in place. For instance, the device in FIG. 2 fits around the underlying bone preventing movement horizontally across the skin of the user. The complement structure of the device allows the device to be easily positioned at the particular location by the patient.

While not illustrated in FIG. 2, the device may also include a spring-like structure to absorb movement vertically and adjust accordingly. For instance, the device may include a layer of foam (e.g., memory foam) adjacent to the skin of the user to absorb movement and adjust the position accordingly, providing a more stable and consistent position of the device against the skin of the user. The foam may also provide an additional benefit of being more comfortable to the user and more adaptive to different bodies.

The embodiment illustrated in FIG. 2 includes a sensor (in the sensor housing 20) in the device 10. The sensor housing can include one or more suitable sensors and may include electronics, circuitry, and/or power supply to support the one or more sensors. Electronics, circuitry, and/or power supply may be located elsewhere in the device in addition to or in alternative to those located in the sensor housing. In some embodiments, the device includes multiple sensor housings each with one or more sensors. The sensor housings may be made integral to the device, while in some embodiments, one or more sensor housings are made physically separateble from the device and may work in conjunction with the device, such as by being alternately mounted to the device, to obtain the physiological measurements.

The circuitry may be mounted on the device, e.g. surface mount device (SMD) components mounted thereon or circuitry printed on or integrated with the device. For instance, the device may comprise a flexible wired board, flexible circuit board or flexible printed circuit board. The device may comprise an analog front-end circuitry (AFE), which may have wires to additional circuitry located elsewhere, such as in another section of the device. The additional circuitry may be a controller, such as a microcontroller (MCU), which can store data from the sensor locally and/or send data offboard, such as for storage and/or processing, through a wireless connection, such as provided by wireless local connectivity or a cellular modem. The additional circuitry may also contain a connector for transferring the data through wired connection, for instance, by real-time streaming or downloading from memory. The same or an alternative connector may also be used for charging the battery, for instance, if a rechargeable battery is used. Wireless charging may also be used as well as non-rechargeable primary cell battery or other power supplies such as various energy harvesting methods. The additional circuitry will be described in greater detail below.

In alternative embodiments, all of the necessary wireless data and power supply may be provided in the sensor housing without connection to additional circuitry in the device.

The sensors may comprise a plurality of the same type of sensor or differing types of sensors and may monitor the user and/or the external environment. Types of sensors that may be used include, but are not limited to: heart rate, heart rate variability, arrhythmia, blood pressure, blood oxygen, blood glucose, humidity, temperature, galvanic skin response and skin moisture or other sensors to monitor the user. In addition or instead of the sensors, a device for taking blood samples from or controlling medicine injection to the user's torso or appendage may be provided.

The sensor and the support electronics/control circuitry may be configured to measure physiological measurements such as heart rate (HR) and heart rate variability (HRV) for example using: electrocardiograph (ECG) methods based on direct contact electrodes on the skin or capacitive contact, opto-electrical photoplethysmography (PPG) measurements using a light source, e.g., a light emitting diode (LED) and photodetector (e.g., transistor/diode or a photodiode (PD)) as a receiver against the skin, LED and photodiode arrays as transmitter-receiver pairs against the skin, a camera as a detector, ultrasonic: Laser Doppler Flowmetry (LDF)/Velocimetry (LDV), radar on the wrist (Nanosecond Pulse Near Field Sensing, NPNS (300 MHz), magnetic methods which utilize Giant-Magneto-Resistance (GMR) to measure Phonocardiographic (PCG) signals, methods utilizing an electrical coil (as a pad of an oscillator), methods utilizing electromechanical film, e.g. Emti(t)-foil, and methods utilizing an acoustic sensor based microphone. In another example embodiment, the sensor and support electronics/control circuity may utilize dynamic light scattering (DLS) in order to measure blood velocity, blood flow and vascular health.

Several artifacts make reliable HR, HRV and peripheral oxygen saturation (SpO2) measurement challenging, such as: movement, breathing and lighting in the environment, for instance, in the case of optical measurements. However, an example embodiment of the location device is configured to reduce the effect of such artifacts and improve the accuracy of HR, HRV and SpO2 measurements.

In some embodiments, the sensor is a reflective photoplethysmogram (PPG) sensor, which may be configured to measure physiological measurements such as heart rate (HR), heart rate variability (HRV), peripheral oxygen saturation (SpO2) and/or regional oxygen saturation (rSO2). A reflective photoplethysmogram (PPG) sensor includes one or several LED(s) and photodetector(s).

For instance, in the embodiment illustrated in FIG. 2, the location device has a structure with a shape that is configured to fit over the underlying bone in the body of the user. Such a location is particularly beneficial when measuring the peripheral oxygen saturation (SpO2). The reflectance from the bone increases the amount of back-reflected signal/data. Large blood vessels are between rib bones and not above rib bones. For sites over the large blood vessels, the SpO2 readings become unreliable when the sensor light probes large light-absorbing objects that move or change diameter with the heartbeat. Thus, by locating the device including the PPG sensor above the bone rather than between bones, the measured SpO2 is more accurate and reliable.

The size of the sensor and other possible electrical components mounted in the sensor housing may vary and can be optimized for the intended use. The size of the location device as well can vary and be optimized for the intended use. The materials for preparing the sensor and sensor housing may vary and may comprise any suitable material such as metal (e.g., stainless steel), plastic (e.g., polyamide), carbon-fiber based materials, or combinations thereof. Dimensions of the components of the device and sensor housing can vary depending on the electrical components, materials, device attachment location, attachment mechanism type, etc. for the embodiment.

The sensor may comprise: electrodes, functional material that converts physical properties into an electrical signal, or other electronics e.g. transistors and passive components, and may be printed onto or integrated into the sensor housing. In certain embodiments, at least one sensor is disposed towards an inwardly facing side of the apparatus, such as facing the side of the apparatus to be disposed adjacent to the skin of a user (e.g., the first surface of the device). The circuitry may be located in the sensor housing or elsewhere in the device. The device may comprise additional electronics, circuitry and a power supply for controlling one or more sensors. The electronics, circuitry and power supply may be located anywhere in the device and may communicate with the sensor by wire or wirelessly. Advantageously, the circuitry is flexible and can be curved so as to conform to any curvature in the apparatus, such as curvature to conform to the shape of the user's torso or appendage to provide a comfortable, wearable apparatus.

The device may be in the shape of a tattoo, patch, band, belt, or strap that can be attached to a user. The device may comprise mechanical support for components of the apparatus, e.g., a user interface, electrical components, control circuitry and power supply for the apparatus, in addition to supporting the circuitry and the sensor. The device may include a mechanical architecture, support means, support member, a frame, a chassis or a skeleton structure which forms the back bone of the apparatus. In some embodiments, the device is configured to at least partially enclose, envelope or wrap around a user's torso or appendage, e.g. a user's wrist, such that the apparatus may be worn by the user around the user's torso or appendage, for example in the form of a belt, band, bracelet or strap for wearing on a wrist or other body location. In other embodiments, the device may be in the form of a patch or similar structure that does not at least partially enclose the torso or an appendage of a user and instead is attached by adhesive or similar means to a portion of a user's body (e.g., the torso or an appendage) or printed on a portion of a user's body, such as by transfer of ink. In some embodiments, the device may have a bracelet/band like form that does not form a closed loop (a "C" shaped cross section). In some embodiments, the device may be provided in a module. As used here "module" refers to a unit or apparatus that excludes certain parts or components that would be added by an end manufacturer or a user.

The size of the location device as well can vary and be optimized for the intended use. The structure of the device may comprise any suitable material and may be rigid, flexible, and/or stretchable substrate. The device may be made of flexible and/or elastic material that allows for bending and shaping of the structure around the user's torso or appendage, such as cloth or bandage material, which may also be breathable, e.g., allow humidity to travel through the structure. For instance, the device may be formed of natural or synthetic materials based on leather, rubber, fabrics, and textiles. The device may alternatively be formed of metal or plastic. The device may comprise a mixture or composite of various materials.

The structure of the device may be configured such that it has portions of varying rigidity. The device may have rigid portions, for instance, to support circuitry, while, in other example embodiments, the device may be substantially flexible. For instance, the incorporation of a spring-like material such as foam enables the device to be flexible and still obtain reliable and consistent readings of the user. The device of an example embodiment is bendable and can provide a more comfortable device for the user to wear.

The device may have flexible portions that may be bent upon user manipulation, e.g., a user squeezing the apparatus between thumb and forefinger, so as to enable movement of the flexible portions of the device. The bending of the device by the user allows the user to conform the shape of the device to the portion of the body to which the device will be adjacent, e.g., the torso or appendage. Such flexibility may improve user comfort. The device may comprise rigid sections, which may be used to house electrical components and hardware mounted therein, to protect delicate circuitry and main electronic components, such as control circuitry of the apparatus. The rigid, bendable, and flexible portions of the device could be achieved by any suitable means, e.g. providing relatively thicker and thinner portions of the device or making such portions of the device from materials having the appropriate mechanical characteristics. Alternatively, a mechanism could be provided to enable bending, such as a hinge.

The device may be provided with means configured to withstand a degree of bending applied by the user to the device or to limit a degree of bending applied by the user. Means configured to withstand a degree of bending of the apparatus or to limit a degree of bending could relate to the selection of materials for parts of the apparatus, e.g., resilient materials, or the structure of the apparatus, e.g., sections of reduced width to permit a degree of bending.

When the apparatus is worn by a user, the first surface 10a of the device corresponds to a side which is proximal to and faces the user and the second surface 10b of the device corresponds to a side which faces away from the user. The first surface 10a may be immediately adjacent to the user's skin and may have a shape that conforms thereto. In other embodiments, the device may comprise additional layers between the first surface and the second surface.

The device may comprise additional sections of circuitry mounted thereon, e.g. SMD components mounted thereon or circuitry printed or integrated thereon, such as a second sensor or an array of sensors. The second sensor may be outwardly disposed on the apparatus so as to be exposed to the external environment (directly exposed or covered by a protective layer) or may be disposed inward to be exposed to the skin of a user. When exposed to the external environment, the second sensor may monitor the external environment, e.g., measure and detect conditions external of the apparatus or other sensors to monitor an external environment (cf. monitoring the user).

The second sensor or array of sensors may comprise a plurality of the same type of sensor or differing types of sensors. For instance, the second sensor or array of sensors may monitor one or more of the following: humidity, temperature, touch, strain, stretch, bend, compression, as well as contortion or user manipulation of the apparatus. A strain sensor may be used to detect user manipulation of a part of the apparatus. For instance, the strain sensor may detect squeezing or flexing of the apparatus. A signal from such a sensor, which may be indicative of user actuation of the apparatus, could be used for a user input or user interface command. The signal may be provided to a controller circuitry mounted on the device and used as an input command for a user interface or to control the apparatus in dependence on detection of user actuation. Advantageously, certain examples of the present disclosure provide a device comprising at least a first sensor optimally arranged to monitor a user of the apparatus, and at least a second sensor optimally arranged to monitor an external environment and/or user actuation.

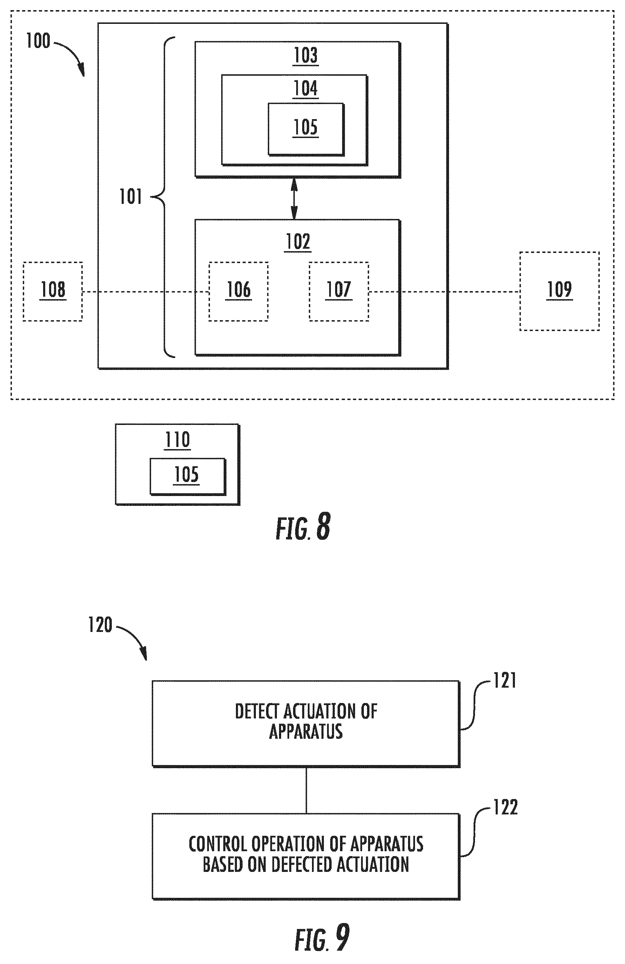

The circuitry may be inherently rigid, e.g., a rigid circuit board, printed circuit board or printed wired board, or may be flexible, e.g., a flexible circuit board, printed circuit board or printed wired board. Advantageously, providing a rigid flat/planar circuitry section may facilitate the mounting and integration of electrical hardware and components, such as a controller as discussed with reference to FIG. 8 or other circuitry, components, devices and sensors (e.g. accelerometers, global positioning system (GPS) receivers), not suitable for printing on flexible circuitry.

In certain embodiments, the location device may comprise an array of integrated/printed sensors thereon so as to substantially cover the inner side of the device thereby increasing the sensing surface area of the device. One or more of the sensors may be configured to monitor a user of the apparatus via contact with a user's torso or appendage, e.g. wrist, (e.g., direct physical contact or contact through a polymer or a glass material) so as to take a physiological measurement of the user. One or more of the sensors may be configured to monitor an external environment. Still further, one or more sensors may be configured to monitor user actuation of the device. The sensors may have one or more protective layers such as polymer material.

Electrical components and electrical hardware, such as a controller, processor and memory, may be mounted and integrated in the device in one embodiment, or may be external to but in communication with the location device of another embodiment. The device of one embodiment may comprise a rigid/flat portion for supporting electronic components, such as interface, power supply, and electronics/circuitry for controlling one or more sensors. The interface may provide power as well as control signals to the plurality of sensors. Also, the interface may receive sensor measurements from the sensors. The interface may provide a wired connection (e.g., via cables or flexible circuitry such as a flexible circuit board (FCB)), a wireless connection (e.g., via radio frequency, near field communications (NFC), infrared (IR) or optical based wireless communication) or alternatively the interface may be via galvanic contacts such as sliding galvanic spring connectors for transferring data and energy to the sensors.

In some embodiments, the sensor housing may be physically separate from the device. FIG. 3 schematically illustrates an example of an apparatus according to example embodiments of the present disclosure. In particular, FIG. 3 provides a cross-sectional view of the apparatus positioned on the user's skin. In the embodiment illustrated in FIG. 3, the device 10 is positioned on the surface 18 of the body 16, which includes bone 14 and tissue 12. The device 10 in FIG. 3 is configured to complement the part of the body where it is intended to be positioned. For instance, the device in FIG. 3 is shaped to fit around the underlying bone 14 in the body 16 of the user. As shown in FIG. 3, in this embodiment, the sensor housing 20 which includes one or more sensors and may also include related circuitry and other electronics is physically separate from the device. In this embodiment, the sensor housing 20 attaches to the device 10 when measurements are needed and is then removed from the device 10 once the measurement is obtained. Various attachment mechanisms for attaching the sensor housing to the device can be used without departing from the intent of the present disclosure. In this embodiment, the sensor housing may be placed along the device when the measurement is needed.

FIG. 4 schematically illustrates an example of an apparatus according to example embodiments of the present disclosure. In particular, FIG. 4 provides a cross-sectional view of the apparatus positioned on the user's skin. The device 10 is positioned on the surface 18 of the body 16 of the user. In the embodiment illustrated in FIG. 4, the sensor housing 20 is physically separate from the device 10. The device 10 includes a window 11 exposing the surface 18 of the body 16 of the user to the environment. In the embodiment illustrated in FIG. 4, the device 10 is positioned on the skin of the user such that the exposed surface 18 of the body 16 is the area for intended physiological measurement and the outline of the device acts as a guide for positioning the sensor housing 20 for measurement of the relevant physiological condition. That is, the sensor housing 20 can be lined-up with the device 10 to position the sensor for obtaining measurements. With the use of a window 11, there is no part of the device blocking the sensor from measuring the physiological condition. For instance, a PPG measurement can be made directly from the skin of the user and not through the device. In such embodiments, the medical professional may apply the device to the user to identify the optimal location for the relevant physiological measurement. The patient can later manually line-up the sensor/sensor housing to the device to locate the optimal location for taking the physiological measurement. While the window 11 illustrated in FIG. 4 has a rectangular shape, the shape of the window and the shape of the device 10 may vary depending on the sensor and/or sensor housing.

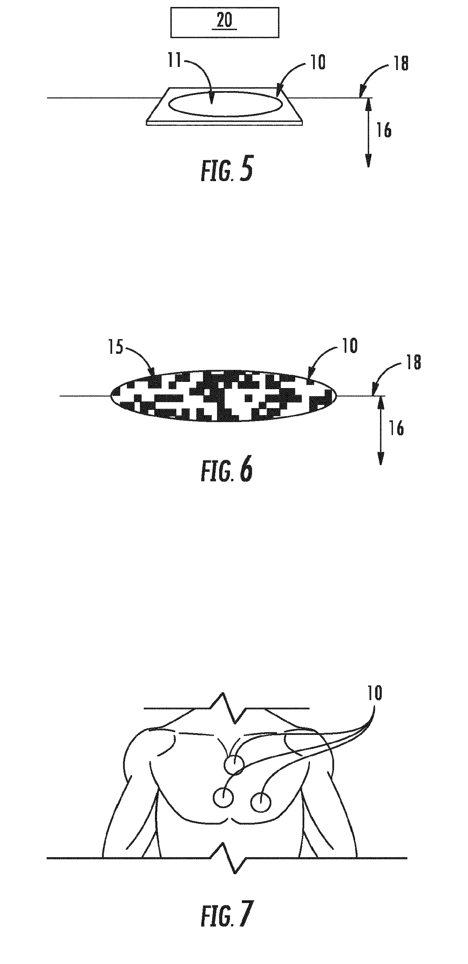

FIG. 5 schematically illustrates an example of an apparatus according to example embodiments of the present disclosure. In particular, FIG. 5 provides a cross-sectional view of the apparatus positioned on the user's skin. The device 10 is positioned on the surface 18 of the body 16 of the user. In the embodiment illustrated in FIG. 5, the sensor housing 20 is physically separate from the device 10. Similar to the device in FIG. 4, the device 10 in FIG. 5 includes a window 11 exposing the surface 18 of the body 16 of the user to the environment. In the embodiment illustrated in FIG. 5, the window 11 is smaller than that seen in FIG. 4 to narrow the focus of the sensor measurement. The device 10 is positioned on the skin of the user such that the exposed surface 18 of the body 16 through the window 11 is the area for intended physiological measurement. The sensor housing 20 may include optical sensor/machine vision to match the sensor to the window 11. That is, the sensor housing 20 can be lined-up with the device 10 by monitoring the exposed and unexposed areas of the skin. For instance, two photodetectors may be used - one which recognizes the exposed/unexposed areas of the skin and a second which takes the physiological measurement. Alternatively, one LED of which intensity is weakened or blocked completely by the tattoo can be used with multiple LEDs that can be used to measure the physiological measurement.

In such embodiments, the medical professional may apply the device to the user to identify the optimal location for the relevant physiological measurement. The patient can later use the device (particularly the exposed and unexposed areas of the device) as a guide for the sensor housing to locate the optimal location for taking the physiological measurement. While the window 11 illustrated in FIG. 4 has a rectangular shape, the shape of the window and the shape of the device 10 may vary depending on the sensor and/or sensor housing, such as the elliptical shape shown in FIG. 5.

FIG. 6 schematically illustrates an apparatus according to example embodiments of the present disclosure. The device 10 is positioned on the surface 18 of the body 16 of the user. In the embodiment illustrated in FIG. 6, the sensor housing 20 is physically separate from the device 10. Similar to the device in FIGS. 4 and 5, the device 10 in FIG. 6 includes an exposed area of the surface 18 of the body 16 of the user to the environment. In the embodiment illustrated in FIG. 6, the device 10 includes identifier 15. The sensor/sensor housing 20 may read the identifier 15 of the device 10. Based on the identifier, information regarding the physiological measurement, the user or the like may be obtained which, in turn, may inform the measurement. In such embodiments and based in part upon the information regarding the physiological measurement, the medical professional may apply the device to the user to identify the optimal location for the relevant physiological measurement and to provide information regarding the user, physiological condition to be measured, and other relevant information. The patient can later use the device as a guide for locating the optimal location for taking the physiological measurement and the identifier 15 can be used to obtain information regarding the physiological measurement when measuring and/or assessing the physiological condition.

The identifier may be a printed code, integrated circuit, or other structure that conveys information to the reader. For instance, the printed code may be a one or two dimensional barcode. The scanning of the information by the sensor housing may relay information regarding the user and/or physiological measurement.

FIG. 7 schematically illustrates a user with an apparatus according to an example embodiment of the present disclosure. In the embodiment illustrated in FIG. 7, the user 17 has a plurality of devices 10 positioned upon the chest of the user 17. In such embodiments, the medical professional may position the devices on the user to identify the optimal location for the relevant physiological measurement. The patient can later use the devices as a guide for locating the optimal location for taking the physiological measurement.

FIG. 8 schematically illustrates an example of a controller for an apparatus according to example embodiments of the present disclosure. The controller controls the sensors and/or other functionality of components in the device. The controller may be integrated in the device or may be separate from, but in communication with the device.

Implementation of the controller 100 can be in hardware alone (e.g. processing circuitry 101 comprising one or more processors 102 and memory circuitry comprising one or more memory elements 103), have certain aspects in software including firmware alone or can be a combination of hardware and software (including firmware).

The controller 100 may be implemented using instructions 105 that enable hardware functionality, for example, by using executable computer program instructions 105 in a general-purpose or special-purpose processor that may be stored on a computer readable storage medium 103 (e.g. memory), to be performed by such a processor.

In the illustrated example, the controller 100 is provided by a processor 102 and memory 103. Although a single processor and a single memory are illustrated in other implementations there may be multiple processors and/or there may be multiple memories some or all of which may be integrated/removable and/or may provide permanent/semi-permanent/ dynamic/cached storage.

In the embodiment illustrated in FIG. 8, the processor 102 is configured to read from and write to the memory 103.

The processor 102 may also comprise an input interface 106 via which data, such as sensor signals and/or commands, are input to the processor 102 from at least one input device 108 (e.g., first and second sensors) and an output interface 107 via which data and/or commands are output by the processor 102 to output device 109. The output device may comprise: a transceiver via which data may be wirelessly communicated to other devices, an audio output device such as a speaker a visual output device such as a display, lights or other visual indication means, or a haptic output device such as a vibrator.

In the embodiment of FIG. 8, the memory 103 comprises a non-transitory computer -readable storage medium that stores a computer program 104 comprising computer program instructions 105 that control the operation of the apparatus when loaded into and executed by the processor 102. The computer program instructions may provide the logic and routines that enable the apparatus to effect functionality, such as controlling the sensors, user input/output, wireless communication and also the method 120 discussed below with respect to FIG. 9.

The computer program instructions 105 may arrive at the controller 100 via any suitable delivery mechanism. The delivery mechanism may be, for example, a non-transitory computer-readable storage medium 110, a computer program product, a memory device, a record medium such as a compact disc read-only memory or digital versatile disc, or an article of manufacture that tangibly embodies the computer program. The delivery mechanism may be a signal configured to reliably transfer the computer program.

FIG. 9 illustrates a method according to example embodiments of the present disclosure. In block 121, user actuation of the location device, or sensor housing, is detected by the processor. For instance, in some embodiments, a second section of flexible circuitry located on the location device or sensor housing could comprise a strain sensor printed thereon configured to detect bending and relative movement of the device. Such an arrangement would be able to recognize user actuation of the device, e.g. a user squeezing the apparatus between his/her thumb and forefinger.

In block 122, operation of the apparatus is controlled by the processor in dependence on the detected actuation. Such control may correspond to a user input or command to effect the operation of the apparatus. For example, the operation may include collection of the measurement data from one or more sensors followed by storage and/or transmission of the measurement data offboard of the location device.

The blocks may represent operations in a method and/or sections of instructions/code 105 in the computer program 104, e.g. such that the controller might be configured to cause the method 120 to be performed.

It will be understood that each block and combinations of blocks, can be implemented by various means, such as hardware, firmware, and/or software including one or more computer program instructions. For example, one or more of the procedures described above may be embodied by computer program instructions 105. In this regard, the computer program instructions which embody the procedures described above may be stored in the memory storage device 103 and performed by the processor 102.

As will be appreciated, any such computer program instructions may be loaded onto a computer or other programmable apparatus (e.g., hardware) to produce a machine, such that the instructions, when performed on the programmable apparatus, create means for implementing the functions specified in the blocks.

The computer program instructions may also be loaded onto a programmable apparatus to cause a series of operations to be performed on the programmable apparatus to produce a computer-implemented process such that the instructions which are performed on the programmable apparatus provide operations for implementing the functions specified in the blocks.

Although examples of the apparatus have been described above, and are further described below in terms of comprising various components, it should be understood that the components may be embodied in or otherwise controlled by a corresponding processing element or processor of the apparatus. In this regard, each of the components described above may be one of more of any device, means or circuitry embodied in hardware, software or a combination of hardware and software that is configured to perform the corresponding functions of the respective components.

Apparatuses in accordance with certain examples of the present disclosure may be configured for: portable use, wearable use (e.g. on a limb portion such as a: wrist, bicep, ankle, etc.) and wired or wireless communication (e.g. via a cellular network, wide area network (WAN) or short range wireless communication protocol).

The apparatus may have additional functions beside communication and comprise: user input interfaces (e.g. buttons, voice control, touch screen, as well as user input by user manipulation of the overall apparatus, e.g. squeezing the apparatus between thumb and forefinger as discussed above) and user output interfaces (e.g. audio-visual and haptic output devices).

In addition to providing sensor measurements and readings, examples of the apparatuses according to the present disclosure may additionally provide one or more audio/text/video communication functions (e.g. tele-communication, video-communication, and/or text transmission (Short Message Service (SMS)/ Multimedia Message Service

(MMS)/emailing) functions), interactive/non-interactive viewing functions (e.g. web-browsing, navigation, television (TV)/program viewing functions), music recording/playing functions (e.g. Moving Picture Experts Group Audio Layer 3 (MP3) or other formats and/or (frequency modulation/amplitude modulation) radio broadcast recording/playing), downloading/sending of data functions, image capture function (e.g. using a (e.g. in-built) digital camera), and gaming functions.

In the above description, the wording "connect," "couple," "communication" and their derivatives mean operationally connected/coupled/in communication. It should be appreciated that any number or combination of intervening components can exist (including no intervening components).

Features described in the preceding description may be used in combinations other than the combinations explicitly described. Although functions have been described with reference to certain features, those functions may be performable by other features whether described or not. Although features have been described with reference to certain examples, those features may also be present in other examples whether described or not.

The term "comprise" is used in this document with an inclusive not an exclusive meaning. That is any reference to X comprising Y indicates that X may comprise only one Y or may comprise more than one Y.

While endeavoring in the foregoing specification to draw attention to those features of the invention believed to be of particular importance it should be understood that the applicant claims protection in respect of any patentable feature or combination of features hereinbefore referred to and/or shown in the drawings whether or not particular emphasis has been placed thereon.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. An apparatus comprising a structure configured to identify a location for a physiological measurement on skin of a user, wherein the structure has a shape configured to correspond to a portion of the user that is in a predefined positional relationship to the location for the physiological measurement such that the structure is configured to engage the portion of the user in order to identify the location for the physiological measurement, wherein the structure comprises a first surface and a second surface, and wherein the first surface is configured to be adjacent to the skin of the user.

2. The apparatus according to claim 1, wherein the structure comprises a layer of ink configured to identify the location for the physiological measurement on the skin of the user.

3. The apparatus according to claim 1, wherein the structure has a shape that is configured to fit over a chest bone of the user.

4. The apparatus according to any of the preceding claims, wherein the structure is configured to connect to a sensor for the physiological measurement.

5. The apparatus according to any of the preceding claims, wherein the structure is configured to be read by a sensor and to convey information regarding the physiological measurement and/or user.

6. The apparatus according to any of the preceding claims, wherein the physiological measurement is one or more of the following: heart rate, heart rate variability, arrhythmia, blood pressure, blood oxygen, blood glucose, humidity, temperature, galvanic skin response, or skin moisture.

7. The apparatus according to any of the preceding claims, wherein the apparatus includes an attachment mechanism configured to attach the structure to the skin of the user.

8. The apparatus according to any of the preceding claims, further comprising one or more of: a sensor, a power supply, electronics, or circuitry.

9. The apparatus according to any one or more of the previous claims, further comprising one or more sensors configured to monitor the user or external environment.

10. The apparatus according to any one or more of the previous claims, further comprising a user actuation section.

11. The apparatus according to any one or more of the previous claims, further comprising at least one sensor configured to detect user actuation.

12. The apparatus according to claim 11, wherein a sensor signal from the at least one sensor configured to detect user actuation is configured to control the apparatus.

13. A method for operating the apparatus of any one or more of the previous claims, the method comprising:
detecting a user actuation of the apparatus; and
controlling operation of the apparatus in dependence on the detected user actuation.

14. A computer program that, when performed by at least one processor, causes the method claimed in claim 13 to be performed.

15. A system comprising the apparatus of any one or more of the previous claims and one or more sensors configured to measure one or more physiological conditions of the user, wherein the one or more sensors is physically separable from the apparatus.
